**Europäisches Patentamt**

**European Patent Office**

**Office européen des brevets**

(11) Veröffentlichungsnummer: **0 062 957**

**A1**

(12) **EUROPÄISCHE PATENTANMELDUNG**

(21) Anmeldenummer: **82200438.8**

(22) Anmeldetag: **10.04.82**

(51) Int. Cl.³: **B 29 C 29/00**
C 08 J 11/04, A 61 L 11/00

(30) Priorität: **10.04.81 NL 8101769**

(43) Veröffentlichungstag der Anmeldung:
**20.10.82 Patentblatt 82/42**

(84) Benannte Vertragsstaaten:
**AT BE CH DE FR GB IT LI LU NL SE**

(71) Anmelder: **STAMICARBON B.V.**
**Postbus 10**
**NL-6160 MC Geleen(NL)**

(72) Erfinder: **Berends, Jan**
**Lynestraat 12**
**NL-6135 EB Sittard(NL)**

(74) Vertreter: **Hoogstraten, Willem Cornelis Roeland et al,**
**OCTROOIBUREAU DSM Postbus 9**
**NL-6160 MA Geleen(NL)**

(54) Verfahren zur Verarbeitung von Kunststoff-Fraktionen aus Hausmüll.

(57) Die Erfindung bezieht sich auf ein Verfahren zur Verarbeitung einer Kunststoff-Fraktion aus Hausmüll, wobei die Kunststoff-Fraktion mit Wasserdampf in Kontakt gebracht wird.

Das erfindungsgemässe Verfahren ist dadurch gekennzeichnet, dass die Kunststoff-Fraktion unterhalb des Erweichungspunktes des Kunststoffs mit Dampf und/oder kochendem Wasser behandelt und danach in wesentlicher Abwesenheit von Wasserdampf geschmolzen und weiterverarbeitet wird.

Die Erfindung ermöglicht, die erheblich störenden Geruchskomponenten aus der aus Hausmüll gewonnenen Kunststoff-Fraktion in befriedigender Weise zu entfernen, und zwar unter Vermeidung der früher auftretenden Probleme im Zusammenhang mit der Verschmutzung der Anlage sowie der schwierigen Weiterverarbeitung des Produkts.

0062957

1

## VERFAHREN ZUR VERARBEITUNG VON KUNSTSTOFF-FRAKTIONEN AUS HAUSMÜLL

Die Erfindung betrifft ein Verfahren zur Verarbeitung von Kunststoff-Fraktionen aus Hausmüll, wobei diese mit Wasserdampf behandelt werden. Ein derartiges Verfahren ist aus der deutschen Offenlegungsschrift 2.229.535 bekannt.

Ein bedeutendes Problem bei der Rückgewinnung von Kunststoff aus Hausmüll besteht in den im Abfallkunststoff vorhandenen Geruchskomponenten. Diese machen sich vor allem nach Verarbeitung der Kunststoff-Fraktion zu einem neuen Endprodukt über die Schmelze, z.B. durch Extrusion, bemerkbar. Bei dem üblichen Verfahren wird eine Geruchsverbesserung des Endprodukts dadurch erreicht, dass man den Kunststoff durch Einleiten des Dampfes eines organischen Lösungsmittels zum Schmelzen bringt und danach zusammenpresst. Dieses Verfahren hat jedoch den Nachteil, dass die zumeist verschiedene Kunststoffsorten enthaltende Kunststoff-Fraktion beim Erhitzen stark klebt, so dass die Anlage in kurzer Zeit erheblich verschmutzt und das ganze Verfahren erschwert wird. Es ist vor allem schwierig, die Schmelzmasse z.B. in einen Extruder oder eine Spritzgiessmaschine zu bringen. Daher begnügt man sich in den meisten Fällen beim üblichen Verfahren mit der Anfertigung eines Pressblockes oder einer Pressplatte. Dies beschränkt die Anwendungsmöglichkeiten des zurückgewonnenen Kunststoffes natürlich in erheblichem Masse.

Die Verarbeitung einer Kunststoff-Fraktion aus Hausmüll erfolgt gemäss der Erfindung, indem die Kunststoff-Fraktion unterhalb des Erweichungspunktes des Kunststoffs mit Dampf und/oder kochendem Wasser behandelt und anschliessend in wesentlicher Abwesenheit von Wasserdampf geschmolzen und weiterverarbeitet wird.
Es ist besonders überraschend, dass es gelungen ist, die Geruchskomponenten in befriedigender Weise zu beseitigen, und zwar bei einer solch niedrigen Temperatur, dass die Kunststoffteile (zumeist Schnitzel und Stücke) im Prinzip formstabil sind. Es entstehen keine Probleme durch Kleben des Kunststoffs im Dampfgefäss, und die Kunststoffteile können nach Abscheiden des restlichen Dampfes sowie Wassers ohne Schwierigkeiten zum Einfülltrichter einer Extrusionsmaschine oder Spritzgiessmaschine geführt werden.

Aus Ausgangsmaterial für das Verfahren gemäss der Erfindung können die Kunststoff-Fraktionen verwendet werden, die an stets mehr Stellen auf verschiedene bekannte Methoden aus Hausmüll abgeschieden werden.

Eine aus Hausmüll erhaltene Kunststoff-Fraktion besteht meist zu einem erheblichen Teil aus Polyäthylen, genauer gesagt Polyäthylen niedriger Dichte mit einer Dichte von ca. 0,91-0,93 $g/cm^3$. Daneben sind in den meisten Fällen andere Polyolefine vorhanden, z.B. Polyäthylen hoher Dichte und Polypropylen sowie andere Kunststoffe, u.a. auch hitzehärtende, wie z.B. Polystyrol, Polyvinylchlorid, ABS-Harz, Phenolharze, Melaminharze, Harnstoffharze usw. Da Polyethylen niedriger Dichte einen niedrigen Erweichungspunkt von ca. 380-390 K hat, erfolgt die erfindungsgemässe Behandlung vorzugsweise bei niedrigen Temperaturen. Ein geeigneter Temperaturbereich ist 330-390 K, vor allem jedoch 360-385 K. Der Druck stellt keine kritische Grösse dar; er kann z.B. 10-1000 kPa betragen. Sehr geeignet ist ein Druck in der Nähe des atmosphärischen Druckes, z.B. 50-200 kPa. Eine geeignete Dampfmenge liegt z.B. zwischen 0,1 und 20 g Dampf pro Gramm Kunststoff pro Stunde, vorzugsweise 0,5-5 g/g.h. Die Dauer der Wasserdampfbehandlung beträgt z.B. 10-200 Min. Grössere Dampfmengen und/oder längere Behandlungszeiten sind möglich, bieten jedoch keine Vorteile.

Es ist zu empfehlen, grössere teile der Kunststoffraktion aus Hausmüll vorher zu zerkleinern - z.B. zu schnitzeln - bis zu einer Maximalgrösse von höchstens 50 mm, vorzugsweise bis höchstens 20 mm.

Nach der Behandlung mit Dampf und/oder kochendem Wasser wird der Kunststoff abgeschieden. Dieser wird vorzugsweise getrocknet und dann in der üblichen Weise extrudiert oder spritzgegossen. Da häufig Bedarf an Granulat besteht, erfolgt nach dem Extrudieren in vielen Fällen Granulierung. Es können jedoch auch direkt Endprodukte hergestellt werden. In beiden Fällen bietet der erheblich verbesserte Geruch des Produktes einen wesentlichen Vorteil.

Die Erfindung wird anhand des folgenden nicht einschränkenden Beispiels näher erläutert.

Beispiel

Eine aus Hausmüll gewonnene Kunststoff-Fraktion wurde geschnitzelt, bis die grösste Abmessung kleiner als 50 mm war. Das

**0062957**

Material wurde über einem Sieb von 1 mm Maschengrösse gesiebt, um kleinere Teile zu entfernen und anschliessend mehrmals mit Leitungswasser bei einer Temperatur von 303 K gewaschen. Das schwimmende Material (d.h. SG unter 1) wurde abgeschieden und bei 348 K getrocknet.

Das auf diese Weise erhaltene Material A wurde 30 Minuten lang bei 383 K und 100 kPa mit Dampf behandelt. Es war keine flüssige Wasserphase anwesend und die Kunststoffschnitzel waren formstabil. Die verwendete Dampfmenge betrug 2 g pro g Kunststoff pro Stunde.

Das mit Dampf behandelte Material wurde schliesslich in Abwesenheit von Dampf geschmolzen, extrudiert und granuliert.

Der Materialgeruch wurde durch eine Beurteilungsgruppe von mindestens vier Personen beurteilt. Die Geruchsbeurteilung der unbehandelten Probe wurde mit Null angenommen; eine positive Beurteilung bedeutet eine Verbesserung, eine negative Beurteilung eine Verschlechterung des Geruchs. Die Ergebnisse der Geruchsproben sind in der Tabelle wiedergegeben.

Zum Vergleich wurden 25 g von Material A bei 303 K 30 Minuten lang in 1 l Wasser verrührt, dem 10 g eines enzymatischen, synthetischen Waschmittels (erhalten unter dem Markennamen 'Dreft') zugesetzt worden waren. Danach wurde der Kunststoff abgeschieden, getrocknet, in Abwesenheit von Wasserdampf geschmolzen, extrudiert und granuliert. Die Resultate der Geruchsproben sind ebenfalls in der Tabelle aufgezeichnet.

Tabelle

| Probe | Behandlung | Geruchsbeurteilung | |
|---|---|---|---|
| | | vor Aufschmelzen | nach Aufschmelzen |
| 1 | keine (blanko) | 0 | 0 |
| 2 | Waschmittel | +3 | −1 |
| 3 | Dampf | +2 | +2 |

Bei Verlängerung der Dampfbehandlung bis zu 180 Minuten (Probe 4) wurden die gleichen Resultate in bezug auf die Geruchsbeurteilung erzielt wie in Probe 3.

Die günstige Geruchsbeurteilung vor Aufschmelzen bei Versuch 2 hängt mit dem im Waschmittel vorhandenen Geruchsverbesserer zusammen. Das Resultat nach Aufschmelzen ist minderwertig.

## PATENTANSPRÜCHE

1. Verfahren zur Verarbeitung einer Kunststoff-Fraktion aus Hausmüll, wobei die Kunststoff-Fraktion mit Wasserdampf in Kontakt gebracht wird, dadurch gekennzeichnet, dass die Kunststoff-Fraktion unterhalb des Erweichungspunktes des Kunststoffs mit Dampf und/oder kochendem Wasser behandelt und danach in wesentlicher Abwesenheit von Wasserdampf geschmolzen und weiterverarbeitet wird.

2. Verfahren nach Anspruch 1, dadurch gekennzeichnet, dass die Wasserdampfbehandlung bei einer Temperatur von 330-390 K durchgeführt wird.

3. Verfahren nach Anspruch 2, dadurch gekennzeichnet, dass die Wasserdampfbehandlung bei einer Temperatur von 360-385 K durchgeführt wird.

4. Verfahren nach einem der Ansprüche 1-3, dadurch gekennzeichnet, dass der Kunststoff vorher zerkleinert wird, bis die grösste Teilchenabmessung maximal 50 mm beträgt.

5. Verfahren nach einem der Ansprüche 1-4, dadurch gekennzeichnet, dass der geschmolzene Kunststoff extrudiert und granuliert wird.

6. Mit Hilfe des Verfahrens nach einem der Ansprüche 1-5 zurückgewonnener Kunststoff.

**0062957**

Nummer der Anmeldung

EP 82 20 0438

## Europäisches Patentamt
## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | DE-A-2 229 535 (KANEGAFUCHI CHEMICAL INDUSTRY) * Seite 4, Zeile 4 - Seite 6, Zeile 4; Seite 8, Zeile 1 - Seite 9, Zeile 24; Seite 14, Zeile 10 - Seite 19, Zeile 12; Seite 24, Zeilen 3-13; Figuren 1-3 * | 1,4-6 | B 29 C 29/00 C 08 J 11/04 A 61 L 11/00 |
| | --- | | |
| A | DE-A-2 621 750 (A. OTTO) * Seite 2, Absatz 4 * | 1 | |
| | --- | | |
| A | DE-A-2 424 158 (AGFA GEVAERT) | | |
| | --- | | |
| A | US-A-4 033 907 (D. WOLF) | | |
| | --- | | RECHERCHIERTE SACHGEBIETE (Int. Cl. 3) |
| A | DE-A-2 550 142 (MUNCHENER MEDIZIN MECHANIK) | | B 29 B C 08 J A 61 L |
| | ----- | | |

Der vorliegende Recherchenbericht wurde für alle Patentansprüche erstellt.

| Recherchenort DEN HAAG | Abschlußdatum der Recherche 07-07-1982 | Prüfer LAVAL J.C.A |
|---|---|---|